# FASCICULE DE BREVET EUROPEEN

(11) **EP 2 346 815 B1**
(45) Date de publication et mention de la délivrance du brevet: **20.04.2016**
(21) Numéro de dépôt: 09783737.1
(22) Date de dépôt: 05.10.2009
(51) Int. Cl.: C07C 253/10, C07C 255/04, B01J 21/00, B01J 23/755

(54) **PROCEDE DE FABRICATION DE COMPOSES COMPRENANT DES FONCTIONS NITRILES**
VERFAHREN ZUR HERSTELLUNG VON VERBINDUNGEN MIT NITRILFUNKLFUNKTIONEN
METHOD FOR PRODUCING COMPOUNDS INCLUDING NITRILE FUNCTIONS

(30) Priorité: 21.10.2008 FR 0805821
(43) Date de publication de la demande: 27.07.2011
(73) Titulaire: Invista Technologies S.à.r.l., 9000 St. Gallen (CH)
(72) Inventeur: MASTROIANNI, Sergio, F-69006 Lyon (FR)
(74) Mandataire: Carpmaels & Ransford LLP
(86) Numéro de dépôt international: PCT/EP2009/062896
(87) Numéro de publication internationale: WO 2010/046226

(56) Documents cités:
- EP-A- 0 336 314
- US-A- 3 496 217
- US-A- 3 864 380
- US-A- 6 048 996
- US-A- 6 127 567

## Description

La présente invention concerne un procédé de fabrication de composés comprenant au moins une fonction nitrile par hydrocyanation d'un composé comprenant au moins une insaturation non conjuguée.

Elle se rapporte plus particulièrement à un procédé de fabrication mettant en oeuvre la réaction du cyanure d'hydrogène avec un composé organique comprenant une insaturation non conjuguée en présence d'un système catalytique comprenant un complexe du nickel à l'état d'oxydation zéro (appelé ci-après Ni(0)) avec au moins un ligand organophosphoré et un cocatalyseur appartenant à la famille des acides de Lewis.

De tels procédés sont connus depuis de nombreuses années et sont exploités industriellement notamment pour la production d'un grand intermédiaire chimique, l'adiponitrile. Ce composé est notamment utilisé dans la fabrication de l'hexaméthylène diamine qui est un monomère important pour la fabrication des polyamides et également un intermédiaire dans la synthèse des composés diisocyanates.

Ainsi, la société DU PONT DE NEMOURS a mis au point et exploité un procédé de fabrication de l'adiponitrile par double hydrocyanation du butadiène. Cette réaction est généralement catalysée par un système catalytique comprenant un complexe du nickel(0) avec des ligands organophosphorés. Ce système comprend également un cocatalyseur, notamment dans l'étape de seconde hydrocyanation, c'est-à-dire hydrocyanation de composés insaturés comprenant une fonction nitrile tels que les pentènenitriles en composés dinitriles.

De nombreux cocatalyseurs ont été proposés dans les brevets et sont généralement des composés appartenant à la famille des acides de Lewis. Un des rôles de ce cocatalyseur ou promoteur est de limiter la production de sous-produits et donc de favoriser la formation de composés de dinitriles linéaires par rapport à la formation de dinitriles ramifiés.

Ainsi, de nombreux halogénures métalliques tels que le chlorure de zinc, le bromure de zinc, le chlorure stanneux, le bromure stanneux ont déjà été proposés, par exemple, dans le brevet US 3 496 217. Le chlorure de zinc est le cocatalyseur préféré.

Il a également été proposé des composés organiques du bore tels que le triphényl de bore ou des composés comprenant deux atomes de bore tels que décrits dans les brevets US 3 864 380 et US 3 496 218, ou des composés organiques de l'étain comme dans le brevet US 4 874 884.

Ces cocatalyseurs ont des propriétés différentes et permettent d'obtenir des sélectivités en dinitriles linéaires tels que l'adiponitrile différentes. Certains de ces cocatalyseurs présentent des inconvénients liés à la difficulté à les extraire du milieu réactionnel ou à la possibilité et facilité d'extraire le système catalytique ou le ligand du nickel (0) en présence de ce cocatalyseur pour le recycler.

Il existe toujours un besoin de trouver de nouveaux cocatalyseurs permettant d'obtenir des sélectivités en dinitriles linéaires de niveaux acceptables et d'utilisation facile.

Un des buts de la présente invention est de proposer une nouvelle famille de cocatalyseurs compatibles et donnant des niveaux de sélectivité en adiponitrile convenables dans la réaction d'hydrocyanation des pentènitriles.

A cet effet, l'invention propose un procédé de fabrication de composés comprenant au moins une fonction nitrile par hydrocyanation d'un composé organique comprenant au moins une insaturation non conjuguée comprenant de 2 à 20 atomes de carbone par réaction avec le cyanure d'hydrogène en présence d'un système catalytique comprenant un complexe du nickel à l'état d'oxydation zéro avec au moins un ligand organophosphoré choisi dans le groupe comprenant les organophosphites, organophosphonites, organophosphinites et organophosphines et un cocatalyseur caractérisé en ce que le cocatalyseur est un composé organométallique répondant à la 35 formule générale I :

[(R)ₐ-(X)_{y}-]ₙM-M₁[-(X)_{z}-(R₁)ₐ₁]ₙ₁

dans laquelle:
M, M₁ identiques ou différents représentent un élément choisi dans le groupe comprenant les éléments suivants : B, Si, Ge, Sn, Pb,
R, R₁ identiques ou différents représentent un radical aliphatique ou un radical comprenant un cycle aromatique ou cycloaliphatique, substitué ou non, pouvant être pontés ou non, ou un radical halogénure,
X représentant un atome d'oxygène, azote, de soufre ou de silicium
y, z sont des nombres entiers identiques ou non égaux à 0 ou 1
n, n₁ sont des nombres entiers égaux à la valence des éléments M, M₁ diminuée de 1
a, a1 sont des nombres entiers identiques ou non égaux à la valence de l'élément X diminuée de 1 si y, z sont égaux à 1, ou égaux à 1 si y, z sont égaux à 0.

Avantageusement R, R₁, identiques ou différents représentent un radical aromatique, aliphatique, cycloaliphatique, substitué ou non, pouvant être pontés ou non, ou un radical halogénure.

Dans la formule ci-dessus, la liaison entre les éléments M et M₁ est symbolisée par une liaison covalente. Toutefois, celle-ci peut être multiple, selon la nature des éléments M et M₁.

Dans la formule ci-dessus, a est égal à la valence de l'élement X diminuée de 1 si y est égal à 1 et a est égal à 1 si y est égal à 0. De même a1 est égal à la valence de l'élement X diminuée de 1 si z est égal à 1 et a1 est égal à 1 si z est égal à 0

Dans la formule ci-dessus, les radicaux R peuvent être identiques ou différents. De même les radicaux R₁ peuvent être identiques ou différents.

Selon une caractéristique préférée de l'invention le cocatalyseur est avantageusement choisi dans le groupe des composés suivants :
- bis(neopentylglycolato)diborane (RN CAS 201733-56-4)
- bis(hexyleneglycolato)diborane (RN CAS 230299-21-5)
- bis(pinacolato)diborane (RN CAS 73183-34-3)
- tetrakis(pyrrolidino)diborane (RN CAS 158752-98-8)
- hexamethyldisilane (RN CAS 1450-14-2)
- tetraphenyldimethyldisilane (RN CAS 1172-76-5)
- diphenyltetramethyldisilane (RN CAS 1145-98-8)
- tris(trimethylsilyl)silane (RN CAS 1873-77-4)
- tetrakis(trimethylsilyl)silane (RN CAS 4098-98-0)
- hexaphenyldisilane (RN CAS 1450-23-3)
- hexamethyldigermane (RN CAS 993-52-2)
- hexaethyldigermane (RN CAS 993-62-4)
- hexaphenyldigermane (RN CAS 2816-39-9)
- hexamethyldistannane (RN CAS 661-69-8)
- hexabutyldistannane (RN CAS 813-19-4)
- hexaphenyldistannane (RN CAS 1064-10-4)
- Triphenylstannyldimethylphenylsilane (RN CAS 210362-76-8)
- triphenylgermanyl-triphenylstannane (RN CAS 13904-13-7)
- hexaphenyldiplumbane (RN CAS 3124-01-4)

Les cocatalyseurs de l'invention sont des composés décrits dans la littérature ainsi que leur procédé de fabrication. Le numéro d'enregistrement RN CAS est donné à titre d'information uniquement La plupart de ces composés sont disponibles commercialement.

Dans un mode préféré de l'invention, le système catalytique de l'invention contient un cocatalyseur conforme à l'invention dans un rapport molaire de cocatalyseur par rapport au nombre d'atomes de nickel compris entre 0.01 et 50 et de préférence entre 0.1 et 10.

Le système catalytique de l'invention comprend un complexe du nickel (0) avec au moins un composé organophosphoré, préférentiellement un composé monodentate tel que le triphénylphosphite ou le tritolylphosphite décrits par exemple, dans les brevets US3496215, DE19953058, FR1529134, FR2069411, US3631191, US3766231, FR2523974 ou un composé bidentate tel que les composés organophosphites décrits dans les brevets WO9906355, WO9906356, WO9906357, WO9906358, WO9952632, WO9965506, WO9962855, US5693843, WO961182, WO9622968, US5981772, WO0136429, WO9964155, WO0213964, US6127567.

Il est également possible d'utiliser des complexes du nickel (0) avec des composés organophoshines monodentates ou bidentates tels que décrits dans les brevets WO02/30854, WO02/053527, WO03/068729, WO04/007435, WO04/007432, FR2845379, WO2004/060855, et plus particulièrement la trithiénylphosphine décrite dans la demande française non publiée n° 0800381 et le DPPX décrit dans le brevet WO2003031392.

De même, le système catalytique de l'invention peut comprendre un complexe du nickel (0) avec des composés organophosphorés monodentates ou bidentates appartenant à la famille des organophosphonites ou organophosphinites.

Il est également possible d'utiliser les cocatalyseurs de l'invention avec un complexe du nickel (0) obtenu avec un mélange de ligand monodentate organophosphite et de ligand bidentate choisi dans les familles de composés appartenant aux organophosphites, organophosphonites, organophosphinites ou organophosphines tels que décrits dans les brevets WO03011457, WO2004/065352.

La description du procédé d'hydrocyanation est réalisée dans plusieurs brevets dont ceux cités précédemment et également dans les articles de C.A. TOLMAN publiés dans les revues Organometallics 3 (1984) 33, Advances in Catalysis (1985) 33-1 et Journal of Chemical Education (1986) vol 63, n°3, pages 199-201.

Brièvement, le procédé de fabrication de composés comprenant au moins une fonction nitrile et plus particulièrement de composés dinitriles tels que l'adiponitrile consiste à faire réagir dans une première étape une dioléfine telle que le 1, 3 butadiène avec du cyanure d'hydrogène, généralement en absence de solvant et en présence d'un système catalytique. La réaction est conduite sous pression pour être en milieu liquide. Les composés nitriles insaturés sont séparés par distillations successives. Les composés nitriles linéaires tels que les pentènenitriles sont alimentés dans une seconde étape d'hydrocyanation.

Avantageusement, les nitriles insaturés non linéaires obtenus en première étape, sont soumis à une étape d'isomérisation pour les transformer en nitriles insaturés linéaires qui sont introduits également dans la seconde étape d'hydrocyanation.

Dans la seconde étape d'hydrocyanation, les nitriles insaturés linéaires sont mis en réaction avec du cyanure d'hydrogène en présence d'un système catalytique.

Les composés dinitriles formés sont séparés par distillations successives après extraction du système catalytique du milieu réactionnel. Plusieurs procédés d'extraction du système catalytique sont décrits, par exemple, dans les brevets US 3 773 809, 4 082 811, 4 339 395 et 5 847 191. Généralement, le système catalytique peut être séparé du milieu réactionnel par décantation en deux phases obtenue par le contrôle des rapports entre les composés mononitriles et les composés dinitriles contenus dans le milieu. Cette séparation peut être améliorée par addition d'ammoniac. Il est également possible de faire précipiter le système catalytique pour le récupérer et le recycler ou d'utiliser un solvant non polaire pour extraire le système catalytique et le séparer des produits nitriles.

Les conditions de température de ces différentes étapes sont comprises entre 10 et 200 °C

Les systèmes catalytiques utilisés dans les première et seconde étapes d'hydrocyanation ainsi que dans l'étape d'isomérisation sont généralement semblables, c'est-à-dire qu'ils contiennent un complexe de nickel (0) identique. Toutefois, le rapport entre le nombre d'atome de nickel et le nombre de molécules de ligand peut être différent dans chacune de ces étapes ainsi que la concentration du système catalytique dans le milieu.

Préférentiellement, le cocatalyseur est présent uniquement dans le système catalytique utilisé pour la seconde étape d'hydrocyanation. Toutefois, il peut être également présent dans l'étape d'isomérisation.

Les caractéristiques et performances du procédé et donc du système catalytique utilisé sont déterminées et illustrées par le taux de transformation (TT) du composé introduit, notamment du mononitrile insaturé introduit dans la seconde étape et par la linéarité en dinitriles linéaires produits, c'est-à-dire le nombre de mole de dinitriles linéaires par rapport au nombre de mole de dinitriles formés. Dans le cas de la fabrication de l'adiponitrile, la linéarité correspond au pourcentage de mole d'adiponitrile (AdN) obtenue par rapport aux nombres de moles de dinitriles formés (AdN + ESN + MGN).

L'invention sera mieux illustrée par les exemples donnés ci-dessous uniquement à titre indicatif, relatifs à la fabrication d'adiponitrile par hydrocyanation de 3-pentènenitrile. Dans ces exemples, le 3-pentènenitrile utilisé est un composé commercialisé par la société Aldrich.

Dans ces exemples, les abréviations suivantes sont utilisées :
- Cod: cyclooctadiène
- 3PN: 3-pentènenitrile
- AdN: Adiponitrile
- ESN: éthylsuccinonitrile
- MGN: méthylglutaronitrile
- TTP: tri-paratolylphosphite
- DPPX: bis(diphénylphosphinométhyl)-1,2-benzène
- TT(Y): taux de transformation du produit à hydrocyaner Y correspondant au rapport du nombre de moles transformées de Y sur le nombre de moles initiales de Y
- Linéarité (L): rapport du nombre de moles d'AdN formées sur le nombre de moles de dinitriles formées (somme des moles d'AdN, ESN et MGN)

Les composés de formule I utilisés dans les exemples ci-dessous sont disponibles commercialement.

### Exemple 1 : Hydrocyanation du 3-PN en AdN

Le mode opératoire général utilisé est le suivant

Sous atmosphère d'argon, dans un tube de verre type Schott de 60 ml équipé d'un bouchon-septum, sont chargés successivement:
- le ligand [5 équivalents molaires de ligand par atome de Ni si le ligand est un monodentate tel que le TTP ou la trithiénylphosphine, ou 2.5 équivalents molaires de ligand par atome de Ni si le ligand est bidentate tel que la DPPX]
- 1.21 g (15 mmol, 30 équivalents) de 3PN anhydre
- 138 mg (0.5 mmol, 1 équivalent) de Ni(Cod)₂
- acide de Lewis (voir les indications dans les tableaux I et II ci-dessous pour la nature et la quantité)

Le mélange est porté sous agitation à 70°C. La cyanhydrine de l'acétone, générateur de HCN, est injectée dans le milieu réactionnel par un pousse-seringue à un débit de 0,45 ml par heure. Après 3 heures d'injection le pousse-seringue est arrêté. Le mélange est refroidi à température ambiante, dilué avec de l'acétone et analysé par chromatographie en phase gazeuse

Les cocatalyseurs utilisés dans les exemples sont listés ci-dessous :
- cocatalyseur A : bis(pinacolato)diborane (RN CAS : 73183-34-3) 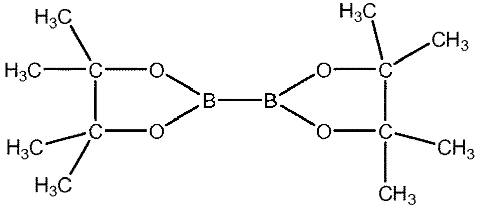
- cocatalyseur B : hexaethyldigermanium(IV) (RN CAS : 993-62-4) 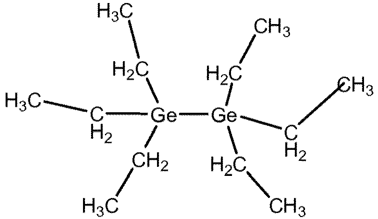
- cocatalyseur C : hexabutyldistannane (RN CAS 813-19-4) 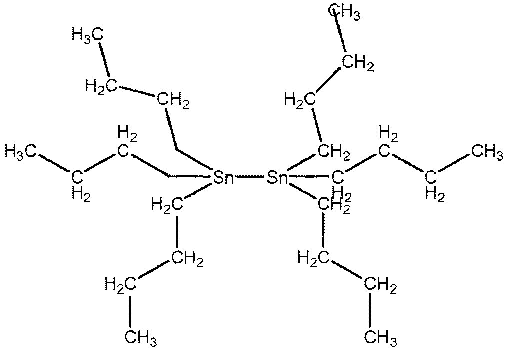
- cocatalyseur D : Diphenyltetramethyldisilane (RN CAS 1145-98-8)

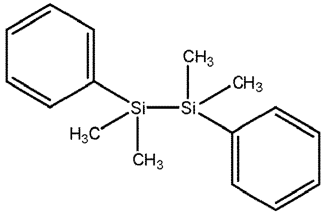

Les résultats sont regroupés dans le tableau I suivant

**Tableau 1: exemples 1 à 11 faisant partie de l'invention**

| exemple | ligand | Acide de Lewis | Ni/Acide de Lewis (molaire) | TT (3PN) | Linéarité |
|---|---|---|---|---|---|
| 1 | TTP | A | 1/1 | 4.8 | 72.8 |
| 2 | TTP | B | 1/1 | 5.7 | 72.4 |
| 3 | TTP | C | 1/1 | 6.2 | 73.2 |
| 4 | TTP | D | 1/0.5 | 1.4 | 67.0 |
| 5 | DPPX | A | 1/0.5 | 17.0 | 93.7 |
| 6 | DPPX | B | 1/0.5 | 19.1 | 74.8 |
| 7 | DPPX | C | 1/0.5 | 21.7 | 82.9 |
| 8 | DPPX | D | 1/0.5 | 22.7 | 79.5 |
| 9 | Tri(2-thiényl)phosphine | A | 1/1 | 14.0 | 69.6 |
| 10 | Tri(2-thiényl)phosphine | B | 1/1 | 17.0 | 76.4 |
| 11 | Tri(2-thiényl)phosphine | C | 1/1 | 32.1 | 82.8 |

## Revendications

1. Procédé de fabrication de composés comprenant au moins une fonction nitrile par hydrocyanation d'un composé organique comprenant au moins une insaturation non conjuguée comprenant de 2 à 20 atomes de carbone par réaction avec le cyanure d'hydrogène en présence d'un système catalytique comprenant un complexe du nickel à l'état d'oxydation zéro avec au moins un ligand organophosphoré choisi dans le groupe comprenant les organophosphites, organophosphonites, organophosphinites et organophosphines et un cocatalyseur **caractérisé en ce que** le cocatalyseur est un composé organométallique répondant à la formule générale I :
[(R)ₐ-(X)_{y}-]ₙM-M₁[-(X)_{z}-(R₁)ₐ₁]ₙ₁
dans laquelle:
M, M₁ identiques ou différents représentent un élément choisi dans le groupe comprenant les éléments suivants : B, Si, Ge, Sn, Pb,
R, R₁ identiques ou différents représentent un radical aliphatique ou un radical comprenant un cycle aromatique ou cycloaliphatique, substitué ou non, pouvant être pontés ou non, ou un radical halogénure,
X représentant un atome d'oxygène, azote, de soufre ou de silicium y, z sont des nombres entiers identiques ou non égaux à 0 ou 1
n, n₁ sont des nombres entiers égaux à la valence des éléments M, M₁ diminuée de 1 a, a₁ sont des nombres entiers identiques ou non égaux à la valence de l'élément X diminuée de 1 si y, z sont égaux à 1, ou égaux à 1 si y, z sont égaux à 0.

2. Procédé de fabrication selon la revendication 1, **caractérisé en ce que** R, R₁ identiques ou différents représentent un radical aromatique, aliphatique, cycloaliphatique, substitué ou non, pouvant être pontés ou non, ou un radical halogénure.

3. Procédé selon la revendication 1 ou 2, **caractérisé en ce que** le cocatalyseur est choisi dans le groupe comprenant les composés suivants :
- bis(neopentylglycolato)diborane
- bis(hexyleneglycolato)diborane
- bis(pinacolato)diborane
- tetrakis(pyrrolidino)diborane
- hexamethyldisilane
- tetraphenyldimethyldisilane
- diphenyltetramethyldisilane
- tris(trimethylsilyl)silane
- tetrakis(trimethylsilyl)silane
- hexaphenyldisilane
- hexamethyldigermane
- hexaethyldigermane
- hexaphenyldigermane
- hexamethyldistannane
- hexabutyldistannane
- hexaphenyldistannane
- triphenylstannyldimethylphenylsilane
- triphenylgermanyl-triphenylstannane
- hexaphenyldiplumbane.

4. Procédé selon l'une de ces revendications 1 à 3, **caractérisé en ce que** le système catalytique comprend un rapport molaire de cocatalyseur par rapport aux moles de Ni compris entre 0.1 et 10.

5. Procédé selon l'une des revendications précédentes, **caractérisé en ce que** le ligand organophosphoré est choisi dans le groupe comprenant les composés organophosphorés monodentate et bidentate.

6. Procédé selon l'une des revendications précédentes, **caractérisé en ce que** les composés organiques à transformer en composés dinitriles sont des composés pentènenitriles.

7. Procédé selon la revendication 6, **caractérisé en ce que** le composé comprenant au moins une fonction nitrile sont l'adiponitrile, le méthylglutaronitrile et le succinonitrile.

## Patentansprüche

1. Verfahren zur Herstellung von Verbindungen mit mindestens einer Nitrilfunktion durch Hydrocyanierung einer organischen Verbindung mit mindestens einer nichtkonjugierten Ungesättigtheit und 2 bis 20 Kohlenstoffatomen durch Reaktion mit Cyanwasserstoff in Gegenwart eines Katalysatorsystems, das einen Komplex von Nickel in der Oxidationsstufe null mit mindestens einem Organophosphorliganden aus der Gruppe umfassend Organophosphite, Organophosphonite, Organophosphinite und Organosphosphine und einen Cokatalysator umfasst, **dadurch gekennzeichnet, dass** es sich bei dem Cokatalysator um eine metallorganische Verbindung der allgemeinen Formel I handelt:
[(Rₐ)-(X)_{y}-]ₙM-M₁[-(X)_{z}- (R₁)ₐ₁]ₙ₁
in der:
M und M₁ gleich oder verschieden sind und für ein Element aus der Gruppe, die die folgenden Elemente umfasst, steht: B, Si, Ge, Sn, Pb,
R und R₁ gleich oder verschieden sind und für einen aliphatischen Rest oder einen Rest mit einem aromatischen oder cycloaliphatischen Ring, der gegebenenfalls substituiert ist und gegebenenfalls verbrückt ist, oder einen Halogenidrest stehen,
X für ein Sauerstoff-, Stickstoff-, Schwefel- oder Siliciumatom steht,
y und z gleiche oder verschiedene Zahlen mit einem Wert von O oder 1 sind, n und n₁ gleiche Zahlen sind, die gleich der um 1 verringerten Wertigkeit der Elemente M und M₁ sind,
a und a₁ gleiche oder verschiedene ganze Zahlen sind, die gleich der um 1 verringerten Wertigkeit des Elements X sind, wenn y und z gleich 1 sind, oder gleich 1 sind, wenn y und z gleich 0 sind.

2. Herstellungsverfahren nach Anspruch 1, **dadurch gekennzeichnet, dass** R und R₁ gleich oder verschieden sind und für einen aromatischen, aliphatischen oder cycloaliphatischen Rest, der gegebenenfalls substituiert ist und gegebenenfalls verbrückt ist, oder einen Halogenidrest stehen.

3. Verfahren nach Anspruch 1 oder 2, **dadurch gekennzeichnet, dass** der Cokatalysator aus der Gruppe ausgewählt wird, die die folgenden Verbindungen umfasst:
- Bis(neopentylglykolato)dibor
- Bis(hexylenglykolato)dibor
- Bis(pinacolato)dibor
- Tetrakis(pyrrolinido)dibor
- Hexamethyldisilan
- Tetraphenyldimethyldisilan
- Diphenyltetramethyldisilan
- Tris(trimethylsilyl)silan
- Tetrakis(trimethylsilyl)silan
- Hexaphenyldisilan
- Hexamethyldigerman
- Hexaethyldigerman
- Hexaphenyldigerman
- Hexamethyldistannan
- Hexabutyldistannan
- Hexaphenyldistannan
- Triphenylstannyldimethylphenylsilan
- Triphenylgermanyltriphenylstannan
- Hexaphenyldibleio

4. Verfahren nach einem der Ansprüche 1 bis 3, **dadurch gekennzeichnet, dass** das Katalysatorsystem ein Molverhältnis von Cokatalysator zu Molen Ni zwischen 0,1 und 10 aufweist.

5. Verfahren nach einem der vorhergehenden Ansprüche, **dadurch gekennzeichnet, dass** der Organophosphorligand aus der Gruppe umfassend einzähnige und zweizähnige Organophosphorverbindungen ausgewählt wird.

6. Verfahren nach einem der vorhergehenden Ansprüche, **dadurch gekennzeichnet, dass** es sich bei den in Dinitrilverbindungen umzuwandelnden organischen Verbindungen um Pentennitrilverbindungen handelt.

7. Verfahren nach Anspruch 6, **dadurch gekennzeichnet, dass** es sich bei den Verbindungen mit mindestens einer Nitrilfunktion um Adiponitril, Methylglutaronitril und Succinonitril handelt.

## Claims

1. Process for producing compounds comprising at least one nitrile function by hydrocyanation of an organic compound comprising at least one nonconjugated unsaturation, comprising from 2 to 20 carbon atoms, by reaction with hydrogen cyanide in the presence of a catalytic system comprising a complex of nickel having the oxidation state of zero with at least one organophosphorus ligand chosen from the group comprising organophosphites, organophosphonites, organophosphinites and organosphosphines and a cocatalyst, **characterized in that** the cocatalyst is an organometallic compound corresponding to general formula I:
**[(R)ₐ-(X)_{y}-]ₙM-M₁[-(X)_{z}-(R₁)ₐ₁]ₙ₁**
in which:
M, M₁, which may be identical or different, represent an element chosen from the group comprising the following elements: B, Si, Ge, Sn, Pb, R, R₁, which may be identical or different, represent an aliphatic radical or a radical comprising an aromatic or cycloaliphatic ring, which is substituted or unsubstituted, and which may or may not be bridged, or a halide radical,
X representing an oxygen, nitrogen, sulphur or silicon atom,
y and z are integers, which may or may not be identical, equal to 0 or 1,
n and n₁ are integers equal to the valency of the elements M, M₁ reduced by 1,
a and a₁ are integers, which may or may not identical, equal to the valency of the element X reduced by 1 if y and z are equal to 1, or equal to 1 if y and z are equal to 0.

2. Production process according to Claim 1, **characterized in that** R and R₁, which may be identical or different, represent an aromatic, aliphatic or cycloaliphatic radical, which is substituted or unsubstituted, and which may or may not be bridged, or a halide radical.

3. Process according to Claim 1 or 2, **characterized in that** the cocatalyst is chosen from the group comprising the following compounds:
- bis(neopentylglycolato)diboron
- bis(hexyleneglycolato)diboron
- bis (pinacolato) diboron
- tetrakis (pyrrolidino) diboron
- hexamethyldisilane
- tetraphenyldimethyldisilane
- diphenyltetramethyldisilane
- tris (trimethylsilyl) silane
- tetrakis(trimethylsilyl)silane
- hexaphenyldisilane
- hexamethyldigermane
- hexaethyldigermane
- hexaphenyldigermane
- hexamethyldistannane
- hexbutyldistannane
- hexaphenyldistannane
- triphenylstannyldimethylphenylsilane
- triphenylgermanyltriphenylstannane
- hexaphenyldilead.

4. Process according to one of these Claims 1 to 3, **characterized in that** the catalytic system comprises a molar ratio of cocatalyst relative to the moles of Ni of between 0.1 and 10.

5. Process according to one of the preceding claims, **characterized in that** the organophosphorus ligand is chosen from the group comprising monodentate and bidentate organophosphorus compounds.

6. Process according to one of the preceding claims, **characterized in that** the organic compounds to be converted to dinitrile compounds are pentenenitrile compounds.

7. Process according to Claim 6, **characterized in that** the compounds comprising at least one nitrile function are adiponitrile, methylglutaronitrile and succinonitrile.
